# EUROPEAN PATENT SPECIFICATION

(11) **EP 2 440 217 B1**
(45) Date of publication and mention of the grant of the patent: **13.12.2017**
(21) Application number: 10727512.5
(22) Date of filing: 07.06.2010
(51) Int. Cl.: A61K 35/20, A61K 35/66, A61P 3/02, A61K 31/195

(54) **NUTRITION FOR IMPROVING MUSCLE STRENGTH IN ELDERLY**
ERNÄHRUNG ZUR VERBESSERUNG DER MUSKELKRAFT IM ALTER
NUTRITION POUR AMÉLIORATION DE LA RÉSISTANCE MUSCULAIRE DES PERSONNES ÂGÉES

(30) Priority: 09.06.2009 WO PCT/NL2009/050319
(43) Date of publication of application: 18.04.2012
(73) Proprietor: N.V. Nutricia, 2712 HM Zoetermeer (NL)
(72) Inventor: Meijer, Erwin Peke, NL-2132 ZV Hoofddorp (NL); Hoijer, Maarten Anne, NL-6814 JA Arnhem (NL)
(74) Representative: Nederlandsch Octrooibureau
(86) International application number: PCT/NL2010/050346
(87) International publication number: WO 2010/143947

(56) References cited:
- WO-A2-02/15720
- US-A1- 2005 153 019

## Description

The invention is in the field of providing nutrition to elderly, particularly for improving muscle strength in elderly.

### BACKGROUND OF THE INVENTION

Old age consists of ages nearing or surpassing the average life span of human beings, and thus the end of the human life cycle. Euphemisms and terms for old people include seniors - chiefly an American usage - or elderly. As occurs with almost any definable group of humanity, some people will hold a prejudice against others - in this case, against old people. This is one form of ageism.

Worldwide, the number of people 65 or older is increasing faster than ever before. Most of this increase is occurring in developed countries. In the United States the percentage of people 65 or older increased from 4 percent in 1900 to about 13 percent in 1998. In 1900, only about 3 million of the nation's citizens had reached 65. By 1998, the number of senior US citizens had increased to about 34 million. Population experts estimate that more than 50 million Americans - about 17 percent of the population - will be 65 or older in 2020. The number of old people is growing around the world chiefly because more children reach adulthood.

Old people have limited regenerative abilities and are more prone to disease, syndromes, and sickness than younger adults. Old people's energy levels decrease, resulting in a decreased level of muscle strength. This decrease in muscular strength then leads to immobility, and decreased cellular insulin sensitivity resulting from a lack of exercise and decreased appetite. This decreased appetite often results in mal- or undernourishment leading to a further decreased muscle strength and so a downward spiral is created. In addition to that, it is postulated that undernourishment leads to damage to the gut epithelium, causing decreased gut mediated immunity, reduced absorption of essential dietary components, also leading to loss of appetite, and thus adding to the downward spiral ultimately leading to loss of muscle strength and immobility.

Therefore, a need exist for a treatment to break this downward spiral.

It is known that as people get older they have reduced levels of friendly bacteria and increased levels of disease-causing bacteria in the gut. At about the age of 60 there is a big drop in bacteria levels, and older people have 1,000-fold less lactic acid (probiotic) bacteria than other younger adults. They are also more susceptible to gastrointestinal infections and bowel conditions, like Irritable Bowel Syndrome.

Malnutrition is remarkably common. It has been estimated that approximately 30% of the world's population suffer from malnutrition in some degree, and it is by no means confined to developing countries. Two studies in the past 10 years showed that nearly 50% of patients admitted to hospitals in Europe and the USA were either malnourished or considered at risk thereof. This condition is well recognized among the elderly. It is commonly found that 7% of those living at home and that are apparently well, fall within the category of being malnourished or considered at risk thereof, and the incidence doubles in subjects aged over 80 years old. Being housebound or ill from additional risk factors.

US 2002/044988 discloses a composition that stimulates body protein synthesis and can improve muscle mass maintenance and recovery. The composition contains a protein source which provides at least about 8 % total calories of the composition and which includes at least about 50 % by weight of whey protein.

WO-2004/026294 discloses the use of nutritional compositions enriched in essential amino acids for tumor induced weight loss and promotion of muscle protein synthesis. Outside the field, RU 2 105 491 discloses a method for preparing a beverage in which granulated sugar is added to diary whey before pasteurization, and wherein fermentation is carried out with ferment prepared from a dry bacterial preparation.

### SUMMARY OF THE INVENTION

The inventors found that the elderly population would benefit from a nutritional treatment capable of increasing the energy levels and muscle strength, and improving the intestinal and immunological functions. The treatment involves the administration of a nutritional product comprising a very high content of whey protein that is supplemented with at least free leucine, preferably with added other free branched chain amino acids (BCAA), probiotic bacteria and prebiotic dietary fibers selected from galactooligosaccharides (GOS), fructooligosaccharides (FOS) and acidic oligosaccharides (AOS), preferably a combination of the three fibers.

One aspect of the invention concerns a composition comprising whey protein, free added BCAA, and probiotic bacteria suitable for stimulating muscle strength in elderly, wherein the composition comprises free BCAA selected from the group consisting of leucine, isoleucine, valine, or a combination thereof, and wherein the composition contains at least 50 g whey protein per 100 g dry weight of the total composition. In a preferred embodiment according to the invention, the composition is a liquid comprising at least 10 weight percent (wt%) dry matter, even more preferably between 12 and 20 wt%. The invention also pertains to the use of such composition for the aforementioned purposes, in elderly.

In another aspect the invention pertains to a composition suitable for elderly, comprising whey protein, free added BCAA, probiotic bacteria and dietary fibers, wherein the composition comprises free BCAA selected from the group Leu, Ileu, Val, or a combination thereof, at least 50 g whey protein per 100 g dry weight of the total composition, and dietary fibers selected from the group consisting of pectin, pectate, alginate, fructans, fructooligosaccharides (FOS) and polysaccharides (e.g. inulin), indigestible dextrins, galactooligosaccharides (GOS), xylooligosaccharides, arabinooligosaccharides, glucooligosaccharides, mannooligosaccharides, fucooligosaccharides and mixtures thereof. The invention also pertains to the use of such composition for the aforementioned purposes, in elderly.

Also, the invention pertains to a composition for use in stimulating muscle strength in elderly, comprising administering elderly with a composition having the aforementioned features. In yet another aspect the invention pertains to the use of the aforementioned composition in the manufacture of a composition/preparation for improving muscle strength, in elderly.

In yet another aspect, the invention pertains to the manufacture of a composition as described above.

### DETAILED DESCRIPTION OF THE INVENTION

### Elderly

The term 'elderly' as used in the present invention means people aged 55 and older.

### Protein/ whey protein

The inventors make use of the inherent differences in the ability of different protein sources to promote (muscle) protein synthesis: Animal protein sources (eg milk, egg, meat) appear more effective than vegetable proteins (eg soy, pea, wheat). Even within these groups, high quality sources like the milk proteins whey and casein show differences in (muscle) protein synthesis. The main differences are:
i. the essential amino acid content of a protein, in particular the leucine content. Specifically, in terms of muscle protein synthesis, the addition of nonessential amino acids to an essential amino acid supplement does not provide an additional stimulatory effect the rate of protein-rich food digestion, splanchnic utilization and subsequent bioavailability of amino acids in the blood circulation. These influence muscle protein synthesis, since muscle protein synthesis is positively related to the blood amino acid concentration. This also underlies the difference between fast- (whey) and slowly-absorbing (casein) proteins.

An important aspect in the elderly that addresses the importance of whey protein in relation to casein protein is that whey protein is more rapidly absorbed, resulting in a 25% higher blood concentration of essential amino acids, than casein that showed only a gradual but prolonged increase of amino acids in the blood. Enhanced protein synthesis following whey protein intake is found compared to the intake of casein protein. The composition according to the invention therefore has high whey protein content.

Preferably whey protein is present in an amount of at least 50 wt% based on dry weight of the composition, more preferably at least 55 weight percent (wt%) and even more preferably between 55 and 75 wt% based on dry weight of the composition. Protein includes all nitrogen sources such as free amino acids, hydrolysates and intact protein.

Compositions can be in liquid or powder form. Preferably the composition is in a liquid form such as - but not limited to - yoghurt drinks, sport drinks, medical nutrition drinks including tube feeds and the like. The high amount of protein in the composition allows low volumes of liquid drink to be used, e.g. between 50 and 100 ml, that still contain an effective amount of protein. This small volume greatly improves the compliance of the product, particularly considering the target group. The amount of protein in the liquid composition is preferably between 6-20 gram per 100 ml even more preferably between 8 and 15 g per 100 ml.

The recommended dietary allowance (RDA) for protein, as promulgated by the Food and Nutrition board of the United States National Academy of Science, is 0.8 g protein/kg body weight/day for adults, regardless of age. This value represents the minimum amount of protein required to avoid progressive loss of lean body mass in most individuals. In order to improve muscle mass, strength and function in elderly, it is preferred to realise protein intake greater than the RDA. In addition, other factors, including immune status, wound healing, blood pressure and bone health may also benefit from the increasing protein intake above the RDA. Furthermore, the RDA does not address the recommended intake of protein in the context of a balanced diet. The inventors believe that concerns about potential detrimental effects of increased protein intake on bone health, renal function, neurological function and cardiovascular function are unfounded in elderly and, on the contrary will be improved in elderly ingesting elevated quantities of protein. Therefore in a preferred embodiment of the invention the composition comprises a relatively high amount of protein, preferably between 35 and 100 energy percent (en%) of the composition is from protein, even more preferably between 50 and 90 en%, and even more preferably between 60 and 90 en% of the composition is from protein. En% protein means the percentage of calories delivered by protein in relation to the total caloric content of the composition. In a most preferred embodiment the protein is a fast absorbing protein such as bovine whey protein, or protein hydrolysates.

### Branched Chain Amino Acids

Branched Chain Amino Acids (BCAA) refer to those amino acids having aliphatic side-chains that are non-linear. These are leucine, isoleucine and valine. The combination of these three essential amino acids make up for approximately 1/3rd of skeletal muscle in the human body, and play an important role in protein synthesis. BCAAs are currently used clinically to aid in the recovery of burn victims, as well as in supplements for providing strength to athletes.

According to the present invention it is preferred that at least free leucine is present in the composition in order to increase the total amount of leucine in the composition. The term 'free amino acid' as used in the present invention means that the amino acid is not part of a protein. Among the essential amino acids, leucine acts both as a substrate for protein synthesis and as a signaling molecule to trigger muscle protein synthesis. The effects of leucine on muscle protein synthesis has been shown in the art (see e.g. Anthony, J.C., et al., Leucine stimulates translation initiation in skeletal muscle of postabsorptive rats via a rapamycin-sensitive pathway J Nutr, 2000. 130(10): p. 2413-9; Katsanos, C.S., et al., A high proportion of leucine is required for optimal stimulation of the rate of muscle protein synthesis by essential amino acids in the elderly Am J Physiol Endocrinol Metab, 2006. 291(2): p. E381-7; Rieu, I., et al., Leucine supplementation improves muscle protein synthesis in elderly men independently of hyperaminoacidaemia. J Physiol, 2006. 575(Pt 1): p. 305-15).

It is preferred to administer a daily dose of at least 1 gram leucine, for stimulating muscle strength. Therefore the composition according to the invention preferably comprises at least 3 gram leucine per 100 g dry weight. The numbers on leucine content are the sum of leucine from protein and added free amino acid. More preferably, the amount of leucine in the composition is between 4.5 and 15 g per 100 g dry weight. In liquid nutrition, leucine preferably amounts to at least 1 g total leucine per 100 ml, more preferably between 1.2 and 5 g total leucine per 100 ml. In practice, if bovine whey protein is incorporated in the composition, between 35 - 70% of leucine should be added as free amino acid. Bovine whey is a preferred protein source since it is rich in BCAA and in particular in leucine.

### Probiotic bacteria

Probiotic bacteria and dietary fibers may be added to the composition to improve intestinal function, e.g. gut motility, barrier function, etc resulting in an improved appetite, therefore synergistically contributing to the effect of high-protein and high-leucine nutrition on muscle strength and mobility of elderly. Probiotics are bacterial components of the normal human intestinal flora, for example lactobacilli and bifidobacteria, that produce as end products of metabolism lactate and short chain fatty acids such as acetate and butyrate. Certain specific probiotic strains have well defined and proven clinical effects for the treatment and/or prevention of diseases of intestinal and extra intestinal origin.

Probiotic bacteria, preferably gram-positive bacteria, are preferably lactic acid bacteria, more preferably bacteria of the genus *Lactobacillus* and/or *Bifidobacterium* and/or *Streptococcus.* It is preferred to employ Gram-positive bacteria comprising at least one *Bifidobacterium* selected from the group consisting of *B.breve, B.infantis, B.bifidum, B.catenulatum, B.adolescentis, B.thermophilum, B.gallicum, B.animalis* or *lactis, B.angulatum, B.pseudocatenulatum, B.thermacidophilum* and *B.longum* more preferably *B. breve, B. infantis, B. bifidum, B. catenulatum, B. longum,* more preferably *B. longum* and *B. breve,* even more preferably *B. breve,* most preferably B. breve I-2219 deposited at the CNCM in Paris, France. Preferably the probiotic bacteria used according to the present invention comprise at least one, more preferably at least two, even more preferably at least three, most preferably at least four different *Bifidobacterium* species.

Preferably the probiotic bacteria used according to the present invention comprise at least one, more preferably at least two *Lactobacillus* species selected from the group consisting of *L. casei, L. reuteri, L paracasei, L. rhamnosus, L. acidophilus, L. johnsonii, L. lactis, L. salivarius, L. crispatus, L. gasseri, L. zeae, L. fermentum* and *L. plantarum,* more preferably *L. casei, L. paracasei, L. rhamnosus, L. johnsonii, L. acidophilus, L. fermentum* and even more preferably *L. paracasei.* Even more preferably the probiotic bacteria used according to the present invention comprise *Bifidobacterium breve* and/or *Lactobacillus paracasei.* In a preferred composition according to the invention the lactic acid bacteria used comprise at least *Bifidobacterium lactis, Lactobacillus acidophilus, Streptococcus thermophilus Prebiotic dietary fibers*

In the context of the invention, prebiotic dietary fibers follow the definition given by the American Association of Cereal Chemists: "the edible parts of plants or similar carbohydrates resistant to digestion and absorption in the human small intestine with complete or partial fermentation in the large intestine." Surprisingly, certain combinations of fibres show better stimulation of NK cell activity. Combinations of galactooligosaccharides (GOS), including trans galactooligosaccharides (TOS), fructooligosaccharides (FOS), including long chain FOS (lcFOS) and short chain FOS (scFOS), and several combinations with acidic oligosaccharides such as pectin hydrolysate all give better effects than the individual fibres.

It was surprisingly found that a mixture of GOS, FOS and pectin hydrolysate had a better NK cell activity stimulating effect than the individual saccharides. As shown in example 4 where it can be seen that a mixture of oligo and polysaccharides stimulate NK T-cell activity in an ageing mouse model. Natural killer T-cell activity is known to be significantly decreased in elderly (Aging and innate immune cells, Journal of Leukocyte Biology Volume 76, 2004). Example 4 shows that in fast ageing mice a specific blend of dietary fibers is capable of stimulating NK cell acitivity, showing that dietary fibers have a potential strong effect in elderly humans. Therefore the composition according to the invention preferably comprises a mixture of two fibres, e.g. GOS and FOS, GOS and pectin hydrolysate, and FOS and pectin hydrolysate and more preferably comprises a mixture of three fibres, e.g. GOS and FOS and pectin hydrolysate.

Preferably the fibres are selected from the group consisting of GOS, FOS and pectin degradation products (e.g. pectin hydrolysates). Even more preferably the ratio of the GOS, FOS and pectin is in the range of approximately 5-9:1:2-10.

Pectin is divided into two main categories: high methoxylated pectin, which is characterized by a degree of methoxylation above 50% and low methoxylated pectin having a degree of methoxylation below 50%. As used herein, "degree of methoxylation" (also referred to as DE or "degree of esterification") is intended to mean the extent to which free carboxylic acid groups contained in the polygalacturonic acid chain have been esterified (e.g. by methylation).

The present acid oligosaccharide is preferably prepared from high methoxylated pectin. Preferably the acid oligosaccharides have a degree of methylation above 20%, preferably above 50 % even more preferably above 70%.

The acid oligosaccharide, preferably pectin hydrolysate, is preferably administered in an amount of between 10 mg and 100 g per day, preferably between 100 mg and 50 g per day, even more between 0.5 g and 20 g per day.

Dietary fibres often have a large molecular weight. The fibres used preferably have an average degree of polymerization of at least 3 and no more than 250. This is because small oligosaccharides do not bind to Toll like receptors and are therefore expected not to be effective in our model systems. This requirement does not imply that no oligosaccharides with DP 1 or 2 may be present. Most commercially available oligosaccharides consist in part of saccharides with a DP 1 and 2, while most have a DP 3 and higher. When calculating weight percentages of the oligosaccharide fraction the weight of all oligosaccharides, including the saccharides with DP 1 and 2 are used in the calculation. The total amount of fibres used in the composition therefore also depends on the source of oligosaccharides and the amount of oligosaccharides with DP 3 or higher present in the source. The maximum DP of about 250 is related to the effect on viscosity. Large molecular weight dietary fibres with a DP higher than about 250 will significantly increase the viscosity of a liquid product resulting in a decreased palatability of the product and possible difficulties during production of a liquid product.

### Calcium

Calcium has a pivotal role in the control of muscle cell action. A decreased availability of calcium in the muscle tissues will inevitably lead to decreased muscle strength. The use of dietary fiber improves the calcium absorption in elderly, and therewith stimulates muscle strength. Calcium intake is often low in elderly due to decreased intake of dairy and other calcium containing products. In a preferred embodiment according to the invention the composition comprises dietary fiber and calcium.

Preferably the amount of dietary fibers in the present composition is between 2 - 10 wt% based on dry weight of the composition.

In a liquid composition preferably 0.2 to 10 g dietary fibers per 100 ml are present. For reasons outlined above, calcium may be present in an amount between 30 and 1000 mg per 100 ml or between 0.05 and 1 wt% based on dry weight of the composition.

### Vitamin E, zinc; other ingredients

Nutrition plays a prominent role in immune response, and the elderly often suffer from malnutrition. Reduced caloric intake is known to slow the aging process and help maintain higher numbers of naive T cells and levels of IL-2. Vitamin E and zinc in particular are important nutrients for the proper functioning of the immune system. Long-term zinc deficiency in the elderly causes a decrease in cytokine production and impaired regulation of helper T cell activity.

Therefore, according to a preferred embodiment the composition according to the invention additionally comprises vitamin E and/or zinc, preferably both.

In a preferred embodiment the composition according to the invention for the stimulation of the immune function and for the stimulation of muscle strength in elderly comprises in addition to high protein at least one selected from; vitamin E, zinc and dietary fibers. More preferably all of these ingredients are present.

### Effect of nutritional composition on the immune system of elderly

Aging affects many components of the immune system, and since the immune system interacts with every organ in the body, a clearer understanding of the immunological changes due to aging is critical for designing effective health care for the elderly. The present inventors now realized that an improved immune function helps to prevent infections and thereby stimulate the mobility of the elderly patients resulting in improved muscle strength.

According to one aspect of the invention the composition according to the invention can be used for
a. increasing muscle mass, strength and function;
b. improving physical capacity and agility; and/or
c. improving vitality, i.e. the ability to perform activities of daily living.

The efficacy of the products can be established in a trial setting by either of the following measures:
- Muscle protein synthesis: incorporation of amino acids is a good indication for muscle strength
- Muscle mass and strength: hand grip and leg strength can be measured
- Physical capacity: can be measured by using an array of different exercise tests
- Activities of Daily Living: can be measured using a score list
- Quality of Life: is normally measured using a questionnaire, score list

### Muscle strength

Muscle strength adds to the quality of life and improved ability to perform activities of daily living. Loss of muscle mass and function in apparently healthy elderly is reflected in the increased difficulty that elderly experience in performing physical activities and activities of daily living, with walking as an obvious example. From 60 years of age loss of muscle mass and strength/function is accelerated and, as a consequence around 30% of muscle strength is lost at the age of 70. When people reach the age of 75 or older it has been demonstrated that the loss of mobility can be significantly impaired:
- 30% can no longer walk a 1 km distance (Lauretani, F., et al., Age-associated changes in skeletal muscles and their effect on mobility: an operational diagnosis of sarcopenia. J Appl Physiol, 2003. 95(5): p. 1851-60)
- Walking (gait) speed declines by 20%
- Activities of Daily Living decline: 50% can no longer lift a weight over 4.5kg (Baumgartner, R.N., et al., Predictors of skeletal muscle mass in elderly men and women. Mech Ageing Dev, 1999. 107(2): p. 123-36.)

To stay independent, people need to maintain or regain physical strength to continue the performance of activities of daily living. Therefore, the term 'muscle strength' as used in the present invention can be measured by 'walking distance', 'walking speed', activity of daily living', 'hand grip strength'.

In one aspect, the invention thus pertains to the use of the composition having the aforementioned characteristics in the manufacture of a preparation for stimulating muscle strength in elderly and/or for treating/preventing malnutrition (thus controlling dietary management) of elderly.

In one aspect, the invention thus pertains to a composition of use in stimulating muscle strength and or preventing/treating malnutrition in a person in need thereof, particularly elderly, by administering the composition having the aforementioned characteristics. In one aspect, the method is non-medical, i.e. non-therapeutic.

The invention also pertains to the non-medical or non-therapeutic use of the composition having the aforementioned characteristics for stimulating muscle strength in elderly and/or for treating/preventing malnutrition (thus controlling dietary management) of elderly

### Method for preparing high protein containing product

The method for preparing the compositions according to the invention involves a careful selection of steps with optimal ranges of the amount of ingredients and strict ranges for pH and temperatures. The most critical for making a fermented, stable product with such high protein content above 8 gram protein per 100 ml, is the pH of the product before emulsifying. This pH preferably is in the range of 3.5 and 4.5 and even more preferably between 3.9 and 4.2. The upper limit of the final product is preferably set at pH 4.2 because at a higher pH the shelf-life of the product becomes problematic. Therefore in a preferred method according to the invention the pH is between 4.0 and 4.2. The effect of pH is demonstrated in Table 1 below.

Another critical step in preparing the compositions according to the invention is the late addition of the free amino acids, i.e. after the fermentation step. The inventors found that during fermentation the probiotic bacteria preferably metabolize these free amino acids leading to a decreased amount of these added amino acids in the end product. Therefore, in the preferred method for preparing the compositions according to the invention, the amino acids are added after the fermentation step.

Therefore, in one aspect the invention pertains to a method for preparing a composition according to the present invention, wherein the method comprises:
a. Mixing whey protein and lactose with demineralized water;
b. adding the bacterial cultures and ferment;
c. adding free amino acids selected from the group consisting of leucine, valine and isoleucine, and optionally add dietary fibers
d. adjusting pH to 3.5 - 4.5, preferably between 4.0 and 4.2;
e. pasteurization;
f. homogenization;
g. if considered appropriate, adjusting pH to between 3.5 and 4.5, preferably between 4.0 and 4.3;
h. optionally adding water to 100%; and
i. sterilizing and packaging product.

**Table 1. Determination of the optimal pH of the product**

| **Product characteristics** | **pH** | | | | | |
|---|---|---|---|---|---|---|
| | **3.84** | **3.93** | **4.05** | **4.09** | **4.22** | **4.33** |
| Particle size distribution D[4,3] | 7.602 | 5.223 | 3.020 | 2..622 | 2.284 | 1.806 |
| Particle size distribution D[0,5] | 6.896 | 5.088 | 2.596 | 2.025 | 1.010 | 0.766 |
| Viscosity (100 l/s) | 32.80 | 32.40 | 22.50 | 20.60 | 19.70 | 17.70 |
| Viscosity (1000 l/s) | 20.00 | 21.90 | 16.90 | 15.70 | 15.10 | 13.80 |
| sediment | 36.88 | 33.43 | 8.61 | 6.97 | 6.90 | 5.99 |

As seen in Table 1, the shelf-life of the product increases when lowering the pH of the product. However, the upper-limit of the pH range is preferably set at pH 4.3 since above this pH microbial growth would make it necessary to sterilize the product for obtaining sufficient shelf-life, which than leads to deterioration of the product characteristics. Although less sediment and lower viscosities can be obtained when increasing the pH, the most optimal pH for this product is selected to be between 4.05 and 4.3. In this pH range the particle size distribution, viscosity and sediment formation are the most acceptable. Similar experiments with casein or other milk protein sources resulted in different optimal pH ranges.

### EXAMPLES

### Example 1. Preferred method for making high protein Leucine containing yogurt

1. Mix whey protein, lactose with demineralized water
2. Pasteurisation step of 15 seconds at 75 degrees Celsius
3. Add and dissolve the bacterial culture
4. Fermentation between 16 and 18 hours
5. Add and dissolve Leucine, valine, isoleucine, dietary fibers and sugar
6. Add and dissolve vitamins and minerals
7. Adjust pH tot 3.5 - 4.5, preferably between 4.0 and 4.2.
8. Add fat and perform inline emulsifying step
9. Pasteurization step of 30s, at 75 degrees Celsius
10. Homogenization at 60 degrees Celsius, 250 -50 bar
11. Adjust to pH 4.2 and add water to 100%
12. Sterilize 30 seconds, at 92 degrees Celsius
13. Package in bottle

### Example 2. Preferred composition fermented product.

| ***Composition*** | ***Per 100 ml*** |
|---|---|
| Protein total (g) | 9.7 |
| Whey protein (g) | 9.0 |
| BCAA total free (g) | 0.7 |
| Leucine free (g) | 0.6 |
| Leucine total (g) | 1.6 |
| Isoleucine free (g) | 0.023 |
| Isoleucine total (g) | 0.6 |
| Valine free (g) | 0.1 |
| Valine total (g) | 0.6 |
| **Fat** (g) | 1.50 |

Rapeseed oil with low erucic acid

### Carbohydrate (g)

| | |
|---|---|
| saccharose | 2.50 |
| Prebiotic fiber (pectin hydrolysate) | 0.20 |

### Vitamins and minerals

| | |
|---|---|
| Calcium (mg) | 200 |
| Zinc | |
| vitamin D | 200IU |
| Vitamin E | |

### Ferment

Probiotic bacteria *Bifidobacterium lactis, Lactobacillus acidophilus, Streptococcus thermophilus*

### Example 3. Sterilized composition with 10 g intact protein per 100ml.

| **Composition** | **Gram per 100 ml** |
|---|---|
| Intact protein | 10.00 |
| Protein total | 10.39 |
| Leucine free | 0.39 |
| Leucine total | 1.40 |
| Isoleucine free | 0.000 |
| Isoleucine total | 0.62 |
| Valine free | 0.00 |
| Valine total | 0.57 |
| fat | 1.50 |
| carbohydrate Dietary fibers | 2.50 |
| GOS/Inulin/AOS | 1.5 |
| Probiotic bacteria | 10⁹-10¹⁰ |
| *Bifidobacterium lactis, Lactobacillus acidophilus, Streptococcus thermophilus* | heat killed CFU |

### Example 4. Stimulation of NK-cell activity with a fiber mix comprising AOS in fast-ageing mice

In order to investigate the effect of fibres on the NK-cell activity in aged mice, the NK-cell activity in splenocytes was measured in 8 months old fast ageing SAMP8 mice that received various doses of scGOS, lcFOS and acid oligosaccharide (AOS) from pectin hydrolysate. The animals were aged on a standard rodent chow, and received the supplemented diets or control diet for the last 43 days ('treatment protocol').

NK cell activity was measured by a modified "JAM" assay. The method as described by P. Matzinger in The JAM test. A simple assay for DNA fragmentation and cell death. J Immunol Methods. 1991 Dec 15;145(1-2):185-92 was used, with the modifications and specifications described here. Yac-1 cells were used as target cells, were labeled for 4-6 hours with 2.5 microCurie/ml tritiated thymidine (Perkin Elmer, Groningen, the Netherlands) and seeded at 5000 cells/well in round-bottom 96-well plates in cell culture medium (RPMI-1640 with 10% fetal calf serum). Murine splenocytes were added to the 96-wellplates as effector cells in three different effector:target (E:T) ratios: 100:1, 50:1 and 25:1. Effector and target cells were incubated for 4 hours at 37 degrees Celsius in an atmosphere containing 5% carbon dioxide. After incubation, the effector and target cells were harvested on Unifilter GF/c plates (Perkin Elmer). These filterplates were dried and counted in a scintillation counter (Wallac Microbeta, Perkin Elmer) and the percentage target cell lysis was calculated as described by P. Matzinger. Statistics have been performed on the separate E:T ratios.

### Results:

At all three ratios, a significant dose-dependent increase in target cell lyses was found using linear regression analysis (slope is significantly greater than zero). The lower three plots show these regressions, statistical testing was performed in SPSS.

The Figure shows an overview of all Effector:Target (E:T) ratios and all GFA doses. It can be seen that the NK-cell activity in the mice significantly increases with the dose of fibres indicating that AOS can actually stimulate the NK cell activity in the aged mice.

## Claims

1. A composition for use in stimulating muscle strength in elderly, comprising bovine whey protein, free branched chain amino acid (BCAA) and probiotic bacteria, wherein the composition comprises one or more free BCAAs selected from the group consisting of leucine, isoleucine and valine, wherein 35-70% of leucine should be added as free amino acid, and at least 50 g whey protein per 100 g dry weight of the total composition.

2. The composition for use according to claim 1, comprising dietary fibers selected from the group of pectin, pectate, alginate, fructan, fructooligosaccharides and fructopolysaccharides, indigestible dextrins, galactooligosaccharides, xylooligosaccharides, arabinooligosaccharides, glucooligosaccharides, mannooligosaccharides and fucooligosaccharides, and mixtures thereof.

3. The composition for use according to claims 1 or 2, wherein the composition comprises between 55 and 75 g whey protein per 100 g dry weight of the total composition.

4. The composition for use according to any one of the proceeding claims, wherein the composition is a liquid comprising at least 10 wt% dry matter, preferably comprising between 12 and 20 wt% dry matter.

5. The composition for use according to claim 3, wherein the composition comprises between 8 and 15 gram protein and a total of at least 1.4 gram leucine per 100 ml.

6. The composition for use according to any one of the preceding claims, wherein at least one of the probiotic bacteria is selected from the group of lactic acid bacteria of the genus *Lactobacillus, Bifidobacterium* and *Streptococcus.*

7. A composition comprising bovine whey protein, free BCAA, probiotic bacteria, and a dietary fiber, wherein the composition comprises one or more free BCAAs selected from the group consisting of leucine, isoleucine and valine, wherein 35-70% of leucine should be added as free amino acid, at least 50 g whey protein per 100 g dry weight of the total composition, and one or more dietary fibers selected from the group of pectins, pectates, alginates, fructans, fructooligosaccharides, indigestible dextrins, galactooligosaccharides, xylooligosaccharides, arabinooligosaccharides, glucooligosaccharides, mannooligosaccharides and fucooligosaccharides.

8. The composition according to claim 7, wherein the composition comprises between 55 and 75 g whey protein per 100 g dry weight of the total composition.

9. The composition according to claims 7 or 8, wherein the composition is a liquid comprising at least 10 wt% dry matter, preferably comprising between 12 and 20 wt% dry matter.

10. The composition according to claim 8, wherein the composition comprises between 8 and 15 gram protein and a total of at least 1.4 gram leucine per 100 ml.

11. The composition according to any one of claims 7-10, wherein at least one of the probiotic bacteria is selected from the group of lactic acid bacteria of the genus *Lactobacillus, Bifidobacterium* and *Streptococcus.*

12. The composition for use according to any one of claims 1 - 6, wherein said elderly suffer from loss of muscle mass and function.

13. The composition according to any one of claims 7-10, for use in the dietary management of malnutrition of elderly.

14. A method for preparing a composition according to any of the claims 1 to 12 comprising:
a. mix whey protein and lactose with demineralized water;
b. add probiotic bacterial cultures and ferment;
c. add free amino acids selected from the group consisting of leucine, valine and isoleucine;
d. optionally add dietary fibers
e. adjust pH to 3.5 - 4.5, preferably between 4.0 and 4.2;
f. pasteurize;
g. homogenize;
h. if appropriate, adjust pH to between 3.5 and 4.5, preferably between 4.0 and 4.3;
i. optionally add water to 100%;
j. sterilize and package product.

## Patentansprüche

1. Eine Zusammensetzung zur Verwendung bei der Stimulation der Muskelkraft bei älteren Menschen, umfassend Rindermolkenprotein, freie verzweigtkettige Aminosäuren (BCAA) und probiotische Bakterien, worin die Zusammensetzung eine oder mehr freie BCAAs umfasst, ausgewählt aus der Gruppe, bestehend aus Leucin, Isoleucin und Valin, worin 35-70% Leucin als freie Aminosäure hinzugefügt werden sollte, und mindestens 50 g Molkenprotein auf 100 g Trockengewicht der Gesamtzusammensetzung.

2. Die Zusammensetzung zur Verwendung nach Anspruch 1, umfassend diätetische Fasern, ausgewählt aus der Gruppe aus Pektin, Pektat, Alginat, Fructan, Fructooligosacchariden und Fructopolysacchariden, unverdaulichen Dextrinen, Galactooligosacchariden, Xylooligosacchariden, Arabinooligosacchariden, Glucooligosacchariden, Mannooligosacchariden und Fucooligosacchariden und Mischungen davon.

3. Die Zusammensetzung zur Verwendung nach Anspruch 1 oder 2, worin die Zusammensetzung zwischen 55 und 75 g Molkenprotein auf 100 g Trockengewicht der Gesamtzusammensetzung umfasst.

4. Die Zusammensetzung zur Verwendung nach einem der vorhergehenden Ansprüche, worin die Zusammensetzung eine Flüssigkeit ist, die mindestens 10 Gew.-% Trockensubstanz, vorzugsweise zwischen 12 und 20 Gew.-% Trockensubstanz umfasst.

5. Die Zusammensetzung zur Verwendung nach Anspruch 3, worin die Zusammensetzung zwischen 8 und 15 Gramm Protein und insgesamt mindestens 1,4 Gramm Leucin auf 100 ml umfasst.

6. Die Zusammensetzung zur Verwendung nach einem der vorhergehenden Ansprüche, worin mindestens eine der probiotischen Bakterien ausgewählt ist aus der Gruppe aus Milchsäurebakterien der Gattung *Lactobacillus, Bifidobacterium* und *Streptococcus.*

7. Eine Zusammensetzung, umfassend Molkenprotein, freie BCAA, probiotische Bakterien und eine diätetische Faser, worin die Zusammensetzung eine oder mehr freie BCAAs, ausgewählt aus der Gruppe, bestehend aus Leucin, Isoleucin, und Valin, umfasst, worin 35-70 Gew.-% Leucin als freie Aminosäure hinzugefügt werden sollte, und mindestens 50 g Molkenprotein auf 100 g Trockengewicht der Gesamtzusammensetzung, und eine oder mehr diätetische Fasern, ausgewählt aus der Gruppe aus Pektinen, Pektaten, Alginaten, Fructanen, Fructooligosacchariden, unverdaulichen Dextrinen, Galactooligosacchariden, Xylooligosacchariden, Arabinooligosacchariden, Glucooligosacchariden, Mannooligosacchariden und Fucooligosacchariden.

8. Die Zusammensetzung nach Anspruch 7, worin die Zusammensetzung zwischen 55 und 75 g Molkenprotein auf 100 g Trockengewicht der Gesamtzusammensetzung umfasst.

9. Die Zusammensetzung nach Anspruch 7 oder 8, worin die Zusammensetzung eine Flüssigkeit ist, die mindestens 10 Gew.-% Trockensubstanz, vorzugsweise zwischen 12 und 20 Gew.-% Trockensubstanz umfasst.

10. Die Zusammensetzung nach Anspruch 8, worin die Zusammensetzung zwischen 8 und 15 Gramm Protein und insgesamt mindestens 1,4 Gramm Leucin auf 100 ml umfasst.

11. Die Zusammensetzung nach einem der Ansprüche 7-10, worin mindestens eine der probiotischen Bakterien ausgewählt ist aus der Gruppe aus Milchsäurebakterien der Gattung *Lactobacillus, Bifidobacterium* und *Streptococcus.*

12. Die Zusammensetzung zur Verwendung nach einem der Ansprüche 1-6, worin die älteren Menschen unter Verlust von Muskelmasse und -funktion leiden.

13. Die Zusammensetzung nach einem der Ansprüche 7-10, zur Verwendung im Ernährungsmanagement von Mangelernährung bei älteren Menschen.

14. Ein Verfahren zur Herstellung einer Zusammensetzung nach einem der Ansprüche 1 bis 12, umfassend:
a. Mischen von Molkenprotein und Laktose mit demineralisiertem Wasser,
b. Zugeben von probiotischen Bakterienkulturen und Fermentieren,
c. Zugeben von freien Aminosäuren, ausgewählt aus der Gruppe, bestehend aus Leucin, Valin und Isoleucin,
d. optional Zugeben von diätetischen Fasern
e. Anpassen des pH auf 3,5-4,5, vorzugsweise zwischen 4,0 und 4,2,
f. Pasteurisieren,
g. Homogenisieren,
h. wenn angezeigt, Anpassen des pH auf zwischen 3,5 und 4,5, vorzugsweise zwischen 4,0 und 4,3,
i. optional Zugeben von Wasser auf 100%,
j. Sterilisieren und Verpacken des Produkts.

## Revendications

1. Composition à utiliser pour stimuler la force musculaire chez les personnes âgées, comprenant de la protéine de lactosérum bovin, un acide aminé à chaîne ramifiée libre (BCAA) et des bactéries probiotiques, dans laquelle la composition comprend un ou plusieurs BCAA libres choisis dans le groupe constitué de leucine, isoleucine et valine, dans laquelle 35-70 % de leucine doivent être ajoutés sous forme d'acide aminé libre, et au moins 50 g de protéine de lactosérum pour 100 g de poids sec de la composition totale.

2. Composition à utiliser selon la revendication 1, comprenant des fibres alimentaires sélectionnées dans le groupe comprenant pectine, pectate, alginate, fructanes, fructooligosaccharides et fructopolysaccharides, dextrines indigestibles, galactooligosaccharides, xylooligosaccharides, arabinooligosaccharides, glucooligosaccharides, mannooligosaccharides et fucooligosaccharides, et des mélanges de ceux-ci.

3. Composition à utiliser selon la revendication 1 ou 2, dans laquelle la composition comprend entre 55 et 75 g de protéine de lactosérum pour 100 g de poids sec de la composition totale.

4. Composition à utiliser selon l'une quelconque des revendications précédentes, dans laquelle la composition est un liquide comprenant au moins 10 % en poids de matière sèche, comprenant de préférence entre 12 et 20 % en poids de matière sèche.

5. Composition à utiliser selon la revendication 3, dans laquelle la composition comprend entre 8 et 15 grammes de protéine et un total d'au moins 1,4 grammes de leucine pour 100 ml.

6. Composition à utiliser selon l'une quelconque des revendications précédentes, dans laquelle au moins l'une des bactéries probiotiques est choisie dans le groupe de bactéries d'acide lactique du genre *Lactobacillus, Bifidobacterium* et *Streptococcus.*

7. Composition comprenant de la protéine de lactosérum bovin, des BCAA libres, des bactéries probiotiques, et une fibre alimentaire, dans laquelle la composition comprend un ou plusieurs BCAA libres choisis dans le groupe constitué de leucine, isoleucine et valine, dans laquelle 35 à 70 % de leucine doivent être ajoutés sous forme d'acide aminé libre, au moins 50 g de protéine de lactosérum pour 100 g de poids sec de la composition totale, et une ou plusieurs fibres alimentaires sélectionnées dans le groupe comprenant pectines, pectates, alginates, fructanes, fructooligosaccharides, dextrines indigestibles, galactooligosaccharides, xylooligosaccharides, arabinooligosaccharides, glucooligosaccharides, mannooligosaccharides et fucooligosaccharides.

8. Composition selon la revendication 7, dans laquelle la composition comprend entre 55 et 75 g de protéine de lactosérum pour 100 g de poids sec de la composition totale.

9. Composition selon la revendication 7 ou 8, dans laquelle la composition est un liquide comprenant au moins 10 % en poids de matière sèche, comprenant de préférence entre 12 et 20 % en poids de matière sèche.

10. Composition selon la revendication 8, dans laquelle la composition comprend entre 8 et 15 grammes de protéine et un total d'au moins 1,4 gramme leucine pour 100 ml.

11. Composition selon l'une quelconque des revendications 7 à 10, dans laquelle au moins l'une des bactéries probiotiques est choisie dans le groupe de bactéries d'acide lactique du genre *Lactobacillus, Bifidobacterium* et *Streptococcus.*

12. Composition à utiliser selon l'une quelconque des revendications 1-6, dans laquelle lesdites personnes âgées souffrent de perte de masse musculaire et de fonction.

13. Composition selon l'une quelconque des revendications 7-10, pour une utilisation dans la prise en charge diététique de la malnutrition de personnes âgées.

14. Procédé de préparation d'une composition selon l'une quelconque des revendications 1 à 12, comprenant les étapes consistant à :
a. mélanger de la protéine de lactosérum et du lactose avec de l'eau déminéralisée ;
b. ajouter des cultures bactériennes probiotiques et faire fermenter ;
c. ajouter des acides aminés libres choisis dans le groupe constitué de leucine, valine et isoleucine ;
d. ajouter éventuellement des fibres alimentaires
e. ajuster le pH à 3,5-4,5, de préférence entre 4,0 et 4,2 ;
f. pasteuriser ;
g. homogénéiser ;
h. le cas échéant, ajuster le pH entre 3,5 et 4,5, de préférence entre 4,0 et 4,3 ;
i. ajouter éventuellement de l'eau jusqu'à 100 % ;
j. stériliser et conditionner le produit.
